# EUROPÄISCHE PATENTSCHRIFT

(11) **EP 1 624 328 B1**
(45) Veröffentlichungstag und Bekanntmachung des Hinweises auf die Patenterteilung: **25.04.2007**
(21) Anmeldenummer: 04024013.7
(22) Anmeldetag: 08.10.2004
(51) Int. Cl.: G02B 6/36, A61B 6/00

(54) **Optischer Drehübertrager mit freiem Innendurchmesser**
Optical rotatable transmitter having a free inside diameter
Transmetteur optique rotatif avec libre diamètre intérieur

(30) Priorität: 02.08.2004 DE 102004037684
(43) Veröffentlichungstag der Anmeldung: 08.02.2006
(73) Patentinhaber: Schleifring und Apparatebau GmbH, 82256 Fürstenfeldbruck (DE)
(72) Erfinder: Schilling, Harry, 85072 Eichstätt (DE)
(74) Vertreter: Lohr, Georg

(56) Entgegenhaltungen:
- WO-A2-03/069392
- DE-C1- 19 543 386
- US-A- 4 555 631

## Beschreibung

### Technisches Gebiet

Die Erfindung betrifft eine Vorrichtung zur Übertragung optischer Signale zwischen gegeneinander drehbaren Einheiten. Derartige Vorrichtungen werden vorzugsweise in Computertomografen eingesetzt.

### Stand der Technik

Zur Übertragung optischer Signale zwischen gegeneinander drehbaren Einheiten, insbesondere mit einem freiem Innendurchmesser sind verschiedene Vorrichtungen bekannt. Grundsätzlich besteht hierin das Problem, ein Mittel zum Transport von Licht entlang des Umfangs der Vorrichtung sowie geeignete Mittel zur ein- und Auskopplung von Licht zu gestalten. Zum Einsatz in Computertomografen müssen derartige Vorrichtungen große freie Innendurchmesser in einer Größenordnung von 1 Meter aufweisen. Die Umfangsgeschwindigkeit bei der Rotation kann in einer Größenordnung von 20 m/s liegen. Gleichzeitig sollten Datenraten mit über 1 Gigabit pro Sekunde (GBaud) möglich sein.

So offenbart die US 4,109,997 einen optischen Drehübertrager, bei dem der Transport von Licht entlang des Umfangs durch Reflexion an zwei gegenüberliegenden Flächen (101, 1) erfolgt. Zur Ein- bzw. Auskopplung von Licht sind Lichtleiter bzw. Glasfasern vorgesehen, wobei die Bündelung bzw. Fokussierung des Lichtstrahls mittels Linsen erfolgt. Eine breitbandige Datenübertragung mit Periodendauern des Modulationssignals, welche wesentlich geringer als die Laufzeit des Signals um den Umfang der Vorrichtung sind, ist nicht möglich, da bei Positionen des Empfängers nahe am Sender ein Mehrwegeempfang von Signalen auftritt. So werden gleichzeitig Signale, die auf kurzem Wege vom Sender empfangen werden sowie gleichzeitig Signale, welche wenigstens einmal um den Umfang der Vorrichtung reflektiert wurden empfangen. Die Laufzeitdifferenz muss klein gegenüber der Periodendauer des Modulationssignals sein. Somit ergibt sich bei einem Innendurchmesser von ca. einem Meter eine Gesamtlaufzeit um den Umfang von ca. 10 Nanosekunden. Dadurch sind beispielsweise bei der Übertragung von digitalen Signalen Bitdauern von maximal 50 Nanosekunden, entsprechend einer maximalen Übertragungsrate von 20 MBaud realisierbar.

Eine Verbesserung des optischen Systems ist in der US 4,525,025 offenbart. So ist darin insbesondere in Fig. 10 ein besonders geeigneter Graben zur Übertragung optischer Signale mit niedrigerer Dämpfung dargestellt. Dieser besteht nur noch aus einem Teil und ist daher kostengünstig herstellbar. Allerdings ist auch in dieser Patentschrift keine wirksame Lösung des Problems der Bandbreitenbegrenzung angegeben. Zudem ist die vorgeschlagene Einkopplung bzw. Auskopplung von Licht durch stumpfe Faserenden nur mit einem äußerst schlechten Wirkungsgrad realisierbar. Somit ist diese Vorrichtung nur für kleine Durchmesser geeignet. Diese Vorrichtung ist äußerst kompakt, sie benötigt aber polarisationserhaltende Faserkoppler, um das Licht eines einzelnen Senders in mehrere Fasern zur Einspeisung aufzuteilen. Derartige

Eine Verbesserung der optischen Ein- bzw. Auskopplung ist in der US 4,555,631 offenbart. Darin erfolgt die Einkopplung optischer Signale in einen verspiegelten Zylinder mittels zweier Spiegel. Zur Auskopplung ist ein zusätzliches Auskoppelelement, welches an einer festen Position im Graben angeordnet ist, vorgesehen. Allerdings ergibt sich auch hier eine hohe Dämpfung der optischen Übertragungsstrecke, da die Einkoppelspiegel insbesondere bei hohen Bewegungsgeschwindigkeiten nicht beliebig nahe an den verspiegelten Zylinder herangeführt werden können. Schließlich ist auch das Problem der Bandbreitenbegrenzung nicht gelöst. So wird das Licht auf zwei Wegen in entgegengesetzten Richtungen von der Einkoppelstelle zur Auskoppelstelle geleitet und schließlich gemeinsam in einem Empfänger ausgewertet. Auch hierbei gilt die Einschränkung, dass die Periodendauer des Modulationssignals wesentlich geringer als die Laufzeit des Lichts um den Umfang der Vorrichtung sein muss. Schließlich muß noch die Kontur des verspiegelten Grabens an die Spiegel angepasst werden.

Eine Vorrichtung mit besonders hohem optischen Wirkungsgrad ist in der US 4,934,783 beschrieben. Darin erfolgt eine Fokussierung des Strahlbündels durch ein Linsensystem. Allerdings ist dieses System sehr aufwändig, teuer in der Herstellung und nur für kleine Durchmesser geeignet. Weiterhin ist auch hier das Bandbreitenproblem nicht gelöst.

In der DE 195 43 386 C1 ist eine Vorrichtung zur breitbandigen Signalübertragung beschrieben, welche zwar eine hohe Bandbreite ermöglicht, aber keinerlei Hinweise auf eine Übertragung mit hoher Übertragungsqualität gibt.

### Darstellung der Erfindung

Der Erfindung liegt die Aufgabe zugrunde, eine relativ kostengünstige Vorrichtung zur Übertragung optischer Signale zwischen zwei gegeneinander drehbaren Einheiten sowie eine Vorrichtung zur Einkopplung optischer Signale in optische Drehübertrager derart zu gestalten, dass eine zuverlässige Übertragung mit niedriger optischer Dämpfung bei großen Durchmessern, hohen mechanischen Bewegungsgeschwindigkeiten und hohen Datenraten ermöglicht wird. Eine weitere Aufgabe der Erfindung ist es, eine solche Vorrichtung derart auszugestalten, dass Signale in definierten Polarisationen übertragen werden. Weiterhin ist die Aufgabe einer besonderen Ausgestaltung der Erfindung, die Vorrichtung derart zu gestalten, dass auch Signale zu übertragen sind, deren Periodendauern klein gegenüber der Ausbreitungsdauer des Lichtes um dem Umfang der Vorrichtung sind.

Eine erfindungsgemäße Lösung dieser Aufgabe ist in den unabhängigen Patentansprüchen angegeben. Weiterbildungen der Erfindung sind Gegenstand der abhängigen Ansprüche.

Die erfindungsgemäße Vorrichtung umfasst einen Lichtleiter, welcher entlang einer Kreisbahn an einer ersten Einheit angeordnet ist. Der Einfachheit halber wird hier nur ein Lichtleiter beschrieben. Selbstverständlich können auch mehrere erfindungsgemäße Anordnungen mit jeweils einem Lichtleiter parallel geschaltet werden. Mit dem Lichtleiter verbunden ist wenigstens ein erster Lichtkoppler zur Einkopplung bzw. Auskopplung von Licht in den Lichtleiter. Mit wenigstens einem dieser ersten Lichtkoppler ist wenigstens ein optischer Sender oder Empfänger verbunden. Ob ein Sender oder Empfänger mit dem Lichtleiter verbunden werden soll wird durch die gewünschte Übertragungsrichtung bestimmt. Soll Licht vom Lichtleiter weg übertragen werden, so ist ein Sender, im anderen Falle ein Empfänger vorzusehen. Zur Informationsübertragung sind die optischen Sender selbstverständlich mit einem Modulationssignal modulierbar.

Weiterhin ist eine zweite Einheit vorgesehen, welche gegenüber der ersten Einheit drehbar gelagert ist. Es wird hier von einer relativen Bewegung der beiden Einheiten gegeneinander ausgegangen und nicht auf drehende bzw. feststehende Einheiten Bezug genommen, da dies ausschließlich eine Frage des Ortsbezugs ist. Dieser zweiten Einheit ist wenigstens ein zweiter Lichtkoppler zugeordnet, der sich mit der Drehung der zweiten Einheit gegenüber der ersten in einer vorgegebenen Bahn bezüglich des Lichtleiters bewegt. Wenigstens einer dieser zweiten Lichtkoppler ist komplementär zum ersten Lichtkoppler wahlweise mit einem optischen Sender oder Empfänger ausgerüstet.

Um nun eine möglichst hohe Bandbreite zu erreichen, ist es notwendig, Licht durch einen ersten Lichtkoppler 4 in zwei in entgegengesetzter Richtung umlaufenden Lichtpfaden in den Lichtleiter 3 einzukoppeln. In einer solchen Anordnung wird bevorzugt gegenüberliegend der Mitte des ersten Lichtkopplers ein Absorber 13 in den Lichtleiter eingefügt, so dass sich zwei gleich lange Zweige des Lichtleiters ergeben. Da sich beim passieren des Absorbers bzw. des Mittelpunkts des ersten Lichtkopplers durch den zweiten Lichtkoppler keine Phasensprünge ergeben sollen, müssen die in den beiden gleich langen Zweigen des Lichtleiters in entgegengesetzter Richtung umlaufenden Signale mit gleicher Phase durch den ersten Lichtkoppler eingespeist werden, so dass sie auch mit gleicher Phase von beiden Seiten im Absorber absorbiert werden können. Diese gleiche Phasenlage wird erreicht durch gleiche optische Weglängen für die beiden optischen Pfade in dem ersten Lichtkoppler. In einem erfindungsgemäßen ersten Lichtkoppler wird Licht von einer Lichtquelle 73 mittels eines optischen Strahlteilers 72 in vorzugsweise zwei gleiche Lichtstrahlen aufgeteilt. Diese werden nun mittels eines ersten Koppelspiegels 70 in einen ersten Lichtpfad 74 und mittels eines zweiten Koppelspiegels 71 in einen zweiten Lichtpfad 75 tangential in zum ersten Lichtpfad entgegengesetzt umlaufender Richtung in den Lichtleiter 3 eingespeist. Hierzu ist der erste Lichtkoppler außerhalb des Radius des Lichtleiters angeordnet. Durch den Einsatz eines Strahlteilers, welcher die Polarisation des Lichts der Lichtquelle 73 erhält, sowie durch die Koppelspiegel 70 und 71 kann Licht in gleicher Polarisation in die beiden Zweige des Lichtleiters eingekoppelt werden. Das hier am Beispiel von zwei Lichtstrahlen vorgestellte Prinzip ist selbstverständlich auch mit 3,4 oder mehreren Lichtstrahlen umsetzbar.

Erfindungsgemäß umfasst der Strahlteiler einen optischen Schalter. Ein solcher Schalter kann derart ausgebildet werden, dass er Licht von der Lichtquelle nur in einen Zweig des Lichtleiters steuert. Dies bedeutet, dass Licht ausschließlich in den Teil des Lichtleiters eingekoppelt wird, in dem sich wenigstens ein zweiter Lichtkoppler 5 zur Auskopplung befindet. Ein solcher optischer Schalter könnte beispielsweise Flüssigkristalle umfassen. Der Vorteil eines optischen Schalters entsprechend der Erfindung ist, dass das Licht von der Lichtquelle 73 nicht in zwei Pfade aufgeteilt werden muss, sondern die gesamte optische Leistung in den jeweiligen Zweig des Lichtleiters 3 eingespeist werden kann in dem sich gerade ein Lichtkoppler 5 befindet.

Eine andere Ausgestaltung der Erfindung sieht vor, dass Licht von einer Lichtquelle 73 durch einen optischen Strahlteiler 72 in zwei Lichtstrahlen aufgeteilt wird. Der erste dieser Lichtstrahlen wird mittels eines ersten Koppelspiegels 70 tangential in den Lichtleiter 3 eingekoppelt. Der zweite Lichtstrahl wird unmittelbar, d. h. ohne Koppelspiegel in den Lichtleiter tangential, in entgegengesetzter Richtung zum ersten Lichtstrahl umlaufend, eingekoppelt. Um hier nur möglichst geringe Phasenverschiebungen zwischen dem ersten und dem zweiten Lichtstrahl zu erzielen, sollte die Anordnung möglichst klein ausgebildet sein.

Eine andere Ausgestaltung der Erfindung sieht Mittel zur hydrostatischen oder hydrodynamischen Lagerung vor. Hierbei wird wenigstens ein zweiter Lichtkoppler 5 mittels eines hydrostatischen oder hydrodynamischen Lagers gegenüber dem Lichtleiter in einer oder zwei Achsen positioniert.

Ein solches hydrostatischen oder hydrodynamisches Lager basiert auf einem dünnen Gasfilm oder Flüssigkeitsfilm, bevorzugt ein Luftfilm zwischen zwei planen Flächen. Der Film weist eine hohe Steifigkeit auf, so dass große Kraftänderungen zu nur geringfügigen Abstandsänderungen führen.

Im Falle eines Gasfilmes wird vorzugsweise ein inertes Gas wie beispielsweise Stickstoff oder bevorzugt ein Edelgas eingesetzt. Das filmbildende Material bzw. das Gas ist vorzugsweise transparent bzw. nicht absorbierend bei der zur optischen Übertragung verwendeten Wellenlänge. Damit verursacht ein Eindringen des Mediums in den Lichtleiter keine Übertragungsstörung. Ebenso kann das Medium gezielt in den Lichtleiter geleitet werden, beispielsweise um diesen von externen Verschmutzungen freizuhalten oder zu reinigen.

Weitere geeignete Medien sind auch Flüssigkeiten, die bei der Betriebstemperatur der Vorrichtung in einen gasförmigen Zustand übergeben. Hiermit ist gerade unter schwierigen Bedingungen gleichzeitig eine Kühlung des Systems möglich.

Bevorzugt erfolgt im Falle eines hydrostatischen Lagers die Speisung des Lagers mittels einer kleinen Pumpe oder eines Druckgasbehälters. Das Medium wird hier zwischen die beiden planen Lagerflächen gedrückt. Da bei derartige Lagern auf Grund des geringen Abstands und der hohen Oberflächengüte der Lagerflächen nur geringste Gas- bzw. Luftmengen verbraucht werden, kann eine solche Speisung mit kostengünstigen Mitteln erfolgen.

Alternativ hierzu kann im Falle eines hydrodynamischen Lagers die Speisung mittels des durch die Bewegung der beiden Einheiten zueinander verursachten Luftstroms erfolgen. In diesem Falle erfolgt die Lagerung durch die Strömung (hydrodynamisches Paradoxon, Bernoulli-Effekt). Hierzu sind vorzugsweise Mittel zur Leitung des durch die Bewegung entstehenden Luftstroms zwischen die Lagerflächen vorgesehen. Im einfachsten Falle bestehen die Luftführungselemente aus einem einfachen Luftleitblech, welches einen Teil der Luftströmungen entsprechend umlenkt. Ebenso sind auch komplexere Ausgestaltungen denkbar, welche beispielsweise zusätzliche Filter enthalten, um den Luftstrom von größeren bzw. kleineren, aber störenden Partikeln zu befreien. Wahlweise können auch Anordnungen gewählt werden, welche beispielsweise für eine weitgehend von der Bewegungsgeschwindigkeit unabhängige Luftgeschwindigkeit sorgen. So kann die Unabhängigkeit der Luftströmungsgeschwindigkeit bei zunehmender Bewegungsgeschwindigkeit durch ein Element, welches für zunehmende Verwirbelung der Luft sorgt, erreicht werden. Ein solches Lager muss selbstverständlich für den Fall niedriger Geschwindigkeiten Notlaufeigenschaften aufweisen. Diese können beispielsweise durch zusätzliche Kombination mit einer hydrostatischen Ausgestaltung erreicht werden.

Besonders günstig ist die Kombination einer hydrodynamischen bzw. hydrostatischen Lagerung in Verbindung mit einer aktiven Lageregelung. Diese können beispielsweise in der gleichen Achse für eine besonders präzise Ausrichtung oder auch in ergänzender Weise in unterschiedlichen Achsen eingesetzt werden. So kann beispielsweise auch die Lageregelung durch Steuerung von Luftstrom bzw. Luftdruck einer hydrodynamischen bzw. hydrostatischen Lagerung erfolgen. Durch diese Kombination erhält man einerseits ein mechanisch robustes System, welches durch eine zusätzliche überlagerte Regelung hochpräzise Eigenschaften erhält. Dadurch können insbesondere auch Toleranzen in dem Abstand der Luftlagerung aufgrund von Temperatur- und Feuchtigkeitsschwankungen der Luft sowie Schwankungen der Geschwindigkeit ausgeglichen werden.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.
Fig. 1 zeigt in allgemeiner Form schematisch einen Teil einer erfindungsgemäßen Vorrichtung.
Fig. 2 zeigt schematisch einen erfindungsgemäßen ersten Koppler.
Fig. 3 und 4 zeigen weitere Ausgestaltungen der Erfindung.

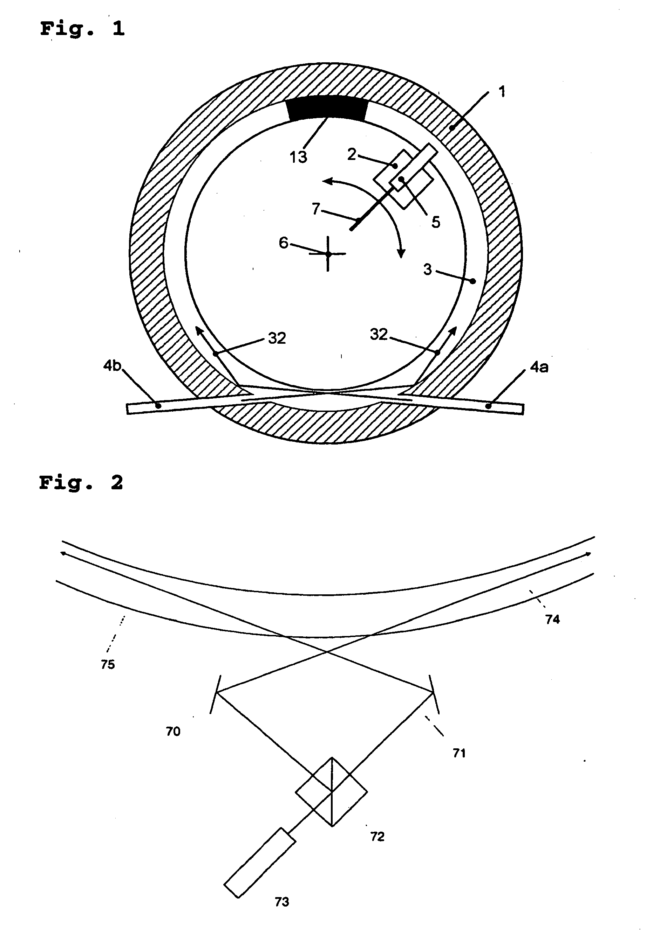

Fig. 1 zeigt in schematischer Form einen Teil einer erfindungsgemäßen Vorrichtung in der Draufsicht. Eine erste Einheit (1) dient zur Aufnahme eines ringförmigen Lichtleiters (3). Dieser Lichtleiter ist beispielsweise ein auf der Innenseite verspiegelter Graben. Eine zweite Einheit (2) dreht sich gegenüber der ersten Einheit um die Drehachse (6). Die zweite Einheit enthält einen zweiten Lichtkoppler (5). Die Funktionsweise soll nun für die beiden Übertragungsrichtungen von der ersten Einheit zur zweiten Einheit bzw. von der zweiten Einheit zur ersten Einheit getrennt dargestellt werden. Übertragung von der ersten Einheit zur zweiten Einheit: Licht aus einem nicht dargestellten Sender wird bezogen auf das Modulationssignal gleichphasig mittels der beiden Teile des ersten Lichtkopplers (4a, 4b) in den Lichtleiter (3) eingespeist. Das Licht vom ersten Lichtkoppler (4a) läuft auf der rechten Seite der Abbildung bis zum Absorber (13). Gleichzeitig läuft das Licht des ersten Lichtkopplers (4b) auf der linken Seite bis zum Absorber (13). Der erste Lichtkoppler (4) ist hier nur schematisch dargestellt. Eine detaillierte Darstellung erfolgt in den folgenden Figuren. Der Absorber ist symmetrisch in Bezug auf die Einkoppelstelle der ersten Lichtkoppler angeordnet, so dass die Lichtwege (32) auf beiden Seiten gleich lang sind. Der Abgriff des Lichts erfolgt mittels eines zweiten Lichtkopplers (5), welcher um die Drehachse (6) entlang der Bahn des Lichtleiters (3) drehbar gelagert ist und das abgegriffene Licht einem optischen Empfänger zuführt. Zur Vereinfachung ist der optische Empfänger ebenfalls nicht abgebildet.

In Figur 2 ist ein erfindungsgemäßer erster Lichtkoppler dargestellt. Das von der Lichtquelle 73 ausgesandte Licht wird in zwei Strahlen durch den Strahlteiler 72 aufgeteilt. Einer dieser Strahlen wird für durch den ersten Koppelspiegel 70 in den ersten Lichtpfad 74 abgelenkt. Der andere Strahl wird über den zweiten Koppelspiegel 71 in den zweiten Lichtpfad 75 abgelenkt. Der zweite Lichtpfad 75 verläuft entgegengesetzt dem ersten Lichtpfad 74. Dadurch wird nun Licht in zwei entgegengesetzten Richtungen in den Lichtleiter 3 eingekoppelt. Das Licht läuft in dem Lichtleiter 3 bis zu dem Absorber 13. Wesentlich für eine breitbandige Signalübertragung ist, dass die beiden Lichtpfade innerhalb des Lichtleiters 3 keine Phasenverschiebung gegeneinander aufweisen. Hierzu muss an der Stelle des ersten Kopplers bzw. der Kreuzung der Lichtstrahlen im Bereich des ersten Kopplers, sowie an der Stelle des Absorber die Phase der beiden Lichtstrahlen gleich sein. Dies ist nur dadurch realisierbar, dass die beiden Lichtpfade im Lichtleiter die gleiche Länge aufweisen und die optischen Pfadlängen von der Lichtquelle 13 bis zur Einkoppelstelle in den Lichtleiter für die beiden Lichtpfade gleich lang sind.

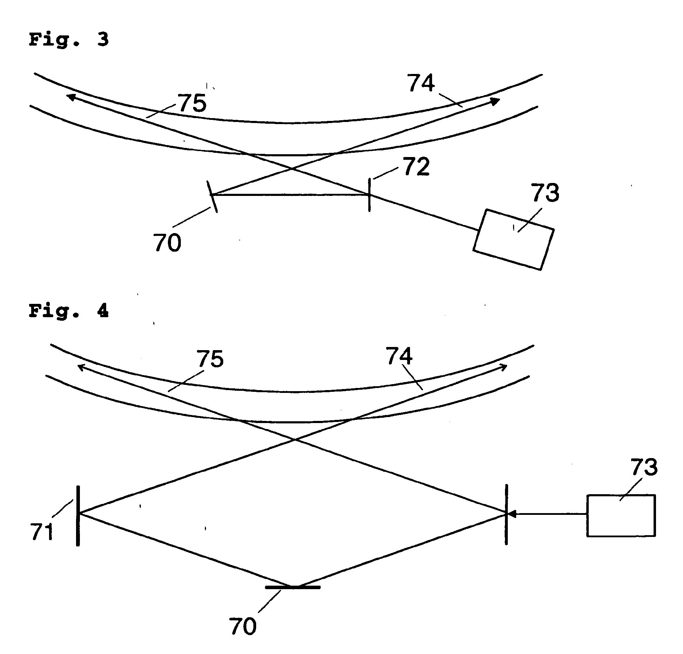

Fig. 3 zeigt eine weitere Ausgestaltung der Erfindung. Das Licht, welches von der Lichtquelle 73 ausgesendet wird, wird in zwei Strahlen durch den Strahlteiler 72 aufgeteilt. Einer dieser Strahlen wird durch den ersten Koppelspiegel 70 in den ersten Lichtstrahl 74 abgelenkt. Der andere Strahl wird direkt in den zweiten Lichtpfad 75 gelenkt.

Eine andere Ausgestaltung der Erfindung ist in Figur 4 dargestellt. Hierbei wird der erste Strahl des Strahlteilers 72 in den ersten Lichtpfad 74 mittels eines ersten Koppelspiegels 70 und eines zweiten Koppelspiegels 71 abgelenkt.

### Bezugszeichenliste

- 1: Erste Einheit
- 2: Zweite Einheit
- 3: Lichtleiter
- 4: Erster Lichtkoppler
- 5: Zweiter Lichtkoppler
- 6: Drehachse der Drehung zwischen erster und zweiter Einheit
- 7: Lichtleitende Faser
- 13: Absorber
- 32: Lichtstrahl
- 70: Erster Koppelspiegel
- 71: Zweiter Koppelspiegel
- 72: Strahlteiler
- 73: Lichtquelle
- 74: Erster Lichtpfad
- 75: Zweiter Lichtpfad

## Patentansprüche

1. Vorrichtung zur Übertragung modulierter optischer Signale zwischen einer ersten Einheit (1) und einer zweiten Einheit (2), wobei die erste Einheit gegenüber der zweiten Einheit drehbar gelagert ist, umfassend
- einen Lichtleiter (3) entlang einer Kreisbahn an der ersten Einheit,
- wenigstens einen mit dem Lichtleiter verbundenen ersten Lichtkoppler (4) zur Lichtein- bzw. Auskopplung in den Lichtleiter,
- wenigstens einen zweiten Lichtkoppler (5), welcher an der zweiten Einheit angeordnet ist, und gegenüber dem Lichtleiter beweglich ist, zur Lichtein- bzw. Auskopplung in den Lichtleiter,
wobei wenigstens ein erster Lichtkoppler (4) außerhalb des Radius des Lichtleiters (3) angeordnet ist und einen ersten Koppelspiegel (70) umfasst, um Licht tangential zum Lichtleiter (3) in einen ersten Lichtpfad (74) einzukoppeln sowie einen zweiten Koppelspiegel (71) umfasst, um Licht in einen, in entgegengesetzter Richtung zum erstem Lichtpfad umlaufenden, zum Lichtleiter (3) tangentialen zweiten Lichtpfad (75) einzukoppeln, und dass weiterhin ein optischer Strahlteiler (72) vorgesehen ist, welcher die optische Energie, die von einer Lichtquelle (73) geliefert wird, dem ersten Koppelspiegel (70) und dem zweiten Koppelspiegel (71) zuführt,
**dadurch gekennzeichnet, dass**
der Strahlteiler (72) wenigstens einen optischen Schalter umfasst, welcher vorgesehen ist, um Licht von der Lichtquelle (73) in einen ausgewählten Lichtpfad zu schalten, in denen sich wenigstens ein zweiter Lichtkoppler befindet.

2. Vorrichtung zur Übertragung modulierter optischer Signale zwischen einer ersten Einheit (1) und einer zweiten Einheit (2), wobei die erste Einheit gegenüber der zweiten Einheit drehbar gelagert ist, umfassend
- einen Lichtleiter (3) entlang einer Kreisbahn an der ersten Einheit,
- wenigstens einen mit dem Lichtleiter verbundenen ersten Lichtkoppler (4) zur Lichtein- bzw. Auskopplung in den Lichtleiter,
- wenigstens einen zweiten Lichtkoppler (5), welcher an der zweiten Einheit angeordnet ist, und gegenüber dem Lichtleiter beweglich ist, zur Lichtein- bzw. Auskopplung in den Lichtleiter, wobei wenigstens ein erster Lichtkoppler einen ersten Koppelspiegel (70) umfasst, um Licht in einen ersten, zum Lichtleiter (3) tangentialen Lichtpfad (74) einzukoppeln und dass weiterhin ein optischer Strahlteiler (72) vorgesehen ist, welcher die optische Energie, die von einer Lichtquelle (73) geliefert wird, in zwei Strahlen teilt, wobei ein Strahl dem ersten Koppelspiegel (70) zugeführt wird,
**dadurch gekennzeichnet, dass**
der zweite Strahl unmittelbar in einen zweiten Lichtpfad (75) tangential zum Lichtleiter (3), entgegengesetzt dem ersten Lichtpfad (74) umlaufend, eingekoppelt wird, und
der Strahlteiler (72) wenigstens einen optischen Schalter umfasst, welcher vorgesehen ist, um Licht von der Lichtquelle (73) in einen ausgewählten Lichtpfad zu schalten, in denen sich wenigstens ein zweiter Lichtkoppler befindet.

3. Vorrichtung nach Anspruch 1 oder 2,
**dadurch gekennzeichnet, dass**
Mittel zur hydrostatischen oder hydrodynamischen Lagerung (12) vorgesehen sind, derart dass wenigstens ein zweiter Lichtkoppler (5) mittels eines flüssigen oder gasförmigen Mediums bzw. einer auf einem flüssigen oder gasförmigen Medium, vorzugsweise Luft basierenden Lagerung gegenüber dem Lichtleiter in einer definierten Position in wenigstens einer Achse, bevorzugt in zwei Achsen senkrecht zur Tangente der Drehbewegung der beiden Einheiten gehalten wird.

## Claims

1. Device for transmitting modulated optical signals between a first unit (1) and a second unit (2), the first unit being supported to be rotatable relative to the second unit, comprising:
- a light guide (3) along a circular track on the first unit;
- at least one first light coupler (4) connected to the light guide for coupling light into or out of the light guide;
- at least one second light coupler (5) disposed on the second unit and movable relative to the light guide for coupling light into and out of the light guide;
in which at least one first light coupler (4) is disposed outside the radius of the light guide (3) and comprises a first coupling mirror (70) for coupling light tangentially to the light guide (3) into a first light path (74), and a second coupling mirror (71) for coupling light into a second light path (75) which extends circumferentially in the opposite direction to the first light path and is tangential to the light guide (3); and furthermore an optical beam splitter (72) is provided for feeding the optical energy supplied by a light source (73) to the first coupling mirror (70) and the second coupling mirror (71);
**characterized in that** the beam splitter (72) comprises at least one optical switch provided for switching light from the light source (73) into a selected light path in which at least one second light coupler is located.

2. Device for transmitting modulated optical signals between a first unit (1) and a second unit (2), the first unit being supported to be rotatable relative to the second unit, comprising:
- a light guide (3) along a circular track on the first unit;
- at least one first light coupler (4) connected to the light guide for coupling light into or out of the light guide;
- at least one second light coupler (5) disposed on the second unit and movable relative to the light guide for coupling light into or out of the light guide;
in which at least one first light coupler (4) comprises a first coupling mirror (70) for coupling light into a first light path (74) tangentially to the light guide (3), and furthermore an optical beam splitter (72) is provided for dividing the optical energy supplied by a light source (73) into two beams, with one beam being supplied to the first coupling mirror (70),
**characterized in that**
the second beam is coupled directly into a second light path (75) tangentially to the light guide (3) to travel in a circumferential direction opposite to that of the first light path (74); and
the beam splitter (72) comprises at least one optical switch provided for switching light from the light source (73) into a selected light path in which at least one second light coupler is located.

3. Device according to claim 1 or 2,
**characterized in that** hydrostatic or hydrodynamic bearing means (12) are provided, such that at least one second light coupler (5) is held relative to the light guide in a defined position along at least one axis, preferably along two axes perpendicular to the tangent to the rotational movement of the two units by means of a liquid or gaseous medium or a bearing means based on a liquid or gaseous medium, preferably air.

## Revendications

1. Dispositif pour transmettre des signaux optiques modulés entre une première unité (1) et une seconde unité (2), la première unité étant montée tournante par rapport à la seconde unité, comprenant
- un guide de lumière (3) le long d'une trajectoire circulaire sur la première unité,
- au moins un premier coupleur de lumière (4) relié au guide de lumière pour le couplage et le découplage de lumière dans le guide de lumière,
- au moins un second coupleur de lumière (5) qui est disposé sur la seconde unité et est mobile par rapport au guide de lumière pour le couplage et le découplage de lumière dans le guide de lumière,
au moins un premier coupleur de lumière (4) étant disposé à l'extérieur du rayon du guide de lumière (3) et comprenant un premier miroir de couplage (70) pour coupler de la lumière tangentiellement au guide de lumière (3) dans un premier chemin de lumière (74) ainsi que comprenant un second miroir de couplage (71) pour coupler de la lumière dans un second chemin de lumière (75) tangentiel au guide de lumière (3), faisant le tour en sens opposé au premier chemin de lumière, et un diviseur de faisceau optique (72) étant prévu en outre, lequel amène au premier miroir de couplage (70) et au second miroir de couplage (71) l'énergie optique qui est fournie par une source de lumière (73),
**caractérisé en ce que**
le diviseur de faisceau (72) comprend au moins un commutateur optique, lequel est prévu pour commuter la lumière de la source de lumière (73) dans un chemin de lumière sélectionné dans lequel se trouve au moins un second coupleur de lumière.

2. Dispositif pour transmettre des signaux optiques modulés entre une première unité (1) et une seconde unité (2), la première unité étant montée tournante par rapport à la seconde unité, comprenant
- un guide de lumière (3) le long d'une trajectoire circulaire sur la première unité,
- au moins un premier coupleur de lumière (4) relié au guide de lumière pour le couplage et le découplage de lumière dans le guide de lumière,
- au moins un second coupleur de lumière (5) qui est disposé sur la seconde unité et est mobile par rapport au guide de lumière pour le couplage et le découplage de lumière dans le guide de lumière,
au moins un premier coupleur de lumière (4) comprenant un premier miroir de couplage (70) pour coupler de la lumière dans un premier chemin de lumière (74) tangentiel au guide de lumière (3) et un diviseur de faisceau optique (72) étant prévu en outre, lequel divise l'énergie optique qui est fournie par une source de lumière (73) en deux faisceaux, un faisceau étant amené au premier miroir de couplage (70),
**caractérisé en ce que**
le second faisceau est couplé directement dans un second chemin de lumière (75) tangentiellement au guide de lumière (3), faisant le tour en sens opposé au premier chemin de lumière (74), et
le diviseur de faisceau (72) comprend au moins un commutateur optique, lequel est prévu pour commuter la lumière de la source de lumière (73) dans un chemin de lumière sélectionné dans lequel se trouve au moins un second coupleur de lumière.

3. Dispositif selon la revendication 1 ou 2,
**caractérisé en ce que**
il est prévu des moyens pour la suspension hydrostatique ou hydrodynamique (12), de façon qu'au moins un second coupleur de lumière (5) soit maintenu, au moyen d'un milieu liquide ou gazeux ou d'une suspension basée sur un milieu liquide ou gazeux, de préférence de l'air, dans une position définie par rapport au guide de lumière sur au moins un axe, de préférence sur deux axes perpendiculairement à la tangente au mouvement de rotation des deux unités.
